Europäisches Patentamt

European Patent Office

Office européen des brevets

(19)

(11) Numéro de publication: **0 515 651 B1**

(12) **FASCICULE DE BREVET EUROPEEN**

(45) Date de publication de fascicule du brevet: **28.06.95**

(51) Int. Cl.6: **C07C 1/08**, C01B 13/18, C01B 13/32

(21) Numéro de dépôt: **92902115.2**

(22) Date de dépôt: **18.12.91**

(86) Numéro de dépôt internationale : **PCT/FR91/01028**

(87) Numéro de publication internationale : **WO 92/11223 (09.07.92 92/17)**

(54) **PROCEDE D'ISOSYNTHESE EN PRESENCE D'UN CATALYSEUR A BASE D'UN OXYDE D'AU MOINS DEUX METAUX DIFFERENTS.**

(30) Priorité: **20.12.90 FR 9016212**

(43) Date de publication de la demande:
**02.12.92 Bulletin 92/49**

(45) Mention de la délivrance du brevet:
**28.06.95 Bulletin 95/26**

(84) Etats contractants désignés:
**BE DE GB NL**

(56) Documents cités:
**EP-A- 0 030 170      DE-A- 2 530 446**
**FR-A- 2 097 253      GB-A- 767 712**
**GB-A- 1 212 347      GB-A- 1 282 307**
**US-A- 4 657 885**

**Chemical Abstracts, vol. 94, no. 4, janvier 1981, (Columbus, Ohio, US), voir page 127, abrégé no. 17846u, & CS,A,182466 (V. MATOUS et al.) 15 mars 1980,voir abrégé**

(73) Titulaire: **INSTITUT FRANCAIS DU PETROLE**
**4, Avenue de Bois Préau**

**F-92502 Rueil-Malmaison (FR)**

Titulaire: **CENTRE NATIONAL DE LA RECHER-CHE SCIENTIFIOUE**
**1, rue Blaise-Pascal,**
**B.P. 296**
**F-67008 Strasbourg Cédex (FR)**

(72) Inventeur: **CHAUMETTE, Patrick**
**34, côte de la Jonchère**
**F-78380 Bougival (FR)**
Inventeur: **ANDRIAMASINORO, Dama**
**2, Boulevard Dostoievsky**
**F-67200 Strasbourg (FR)**
Inventeur: **KIEFFER, Roger**
**39, rue de Chambord**
**F-67200 Strasbourg (FR)**
Inventeur: **KIENNEMANN, Alain**
**30, rue des Cottages**
**F-67400 Illkirch (FR)**
Inventeur: **POIX, Paul**
**61, rue de Lorraine**
**F-67380 Lingolsheim (FR)**

EP 0 515 651 B1

Inventeur: **REHSPRINGER, Jean-Luc**
**2, rue du Poumon**
**F-67000 Strasbourg (FR)**

(74) Mandataire: **Andreeff, François et al**
**INSTITUT FRANCAIS DU PETROLE**
**4, avenue de Bois-Préau**
**F-92506 Rueil-Malmaison Cédex (FR)**

## Description

La présente invention concerne un procédé d'isosynthèse en présence d'un catalyseur à base d'un oxyde d'au moins deux métaux différents, ledit oxyde ayant une surface spécifique d'au moins 20 $m^2/g$ et une structure de type cubique, perovskite, grenat, ilménite ou fluorine ou de type apparenté au type fluorine. Ce type de catalyseur est décrit dans GB-A-767712.

L'invention se caractérise également par la préparation du catalyseur.

Des procédés de préparation d'oxydes de plusieurs métaux ayant une structure de l'un des types sus-mentionnés ont déjà été décrits dans l'art antérieur, mais ils conduisent généralement à des oxydes ayant une surface spécifique (mesurée par la méthode classique B.E.T.) inférieure à 10 $m^2/g$, car il est nécessaire d'effectuer un traitement thermique à très haute température (au-delà de 1000°C) pour obtenir un oxyde présentant une structure de l'un des types précédents (D. MICHEL, M. PEREZ Y JORBA, R. COLLONGUES, Mater. Res. Bull. 9(11), 1457, 1974).

Le procédé de préparation décrit dans la demande de brevet GB-A-1282307 permet d'obtenir des oxydes de plusieurs éléments sous forme divisée à l'aide d'une substance organique fortement complexante et décomposable à la chaleur par concentration, déshydratation puis décomposition d'une solution contenant des sels métalliques et l'agent complexant en évitant toute précipitation. Mais les produits obtenus par ce procédé sont susceptibles de s'hydrater ou/et de se carbonater en présence d'eau ou/et de dioxyde de carbone.

Un des buts de la présente invention est de pallier aux inconvénients sus-mentionnés en obtenant, par un procédé de préparation particulier, des oxydes d'au moins deux métaux différents, ayant une surface spécifique d'au moins 20 $m^2/g$ et une réactivité sensiblement nulle vis-à-vis de l'eau et du dioxyde de carbone.

Selon l'invention, pour le procédé d'isosynthèse, il est maintenant proposé un procédé de préparation d'un oxyde d'au moins deux métaux différents, l'un au moins desdits métaux étant choisi parmi les métaux des groupes IIa et IIIb de la classification périodique des éléments, l'autre au moins desdits métaux étant choisi parmi les métaux des groupes Ia, IIa, IIIb, IVb, Vb, VIb, IIIa et IVa de la classification périodique des éléments, ledit oxyde ayant une surface spécifique d'au moins 20 $m^2/g$ et une structure de l'un des types suivants : cubique, perovskite, grenat, ilménite, fluorine ou type apparenté au type fluorine (par exemple pyrochlore), ledit procédé étant caractérisé en ce qu'il comprend les étapes suivantes :

1) un sel ou un oxyde simple de chacun desdits métaux est mis en solution dans un solvant approprié (un oxyde simple est un oxyde d'un seul métal),
2) les solutions, contenant chacune l'un desdits métaux, obtenues à l'étape 1) sont mélangées ensemble,
3) le mélange de solutions obtenu à l'étape 2) est évaporé jusqu'à l'obtention d'un gel contenant lesdits métaux,
4) le gel obtenu à l'étape 3) est calciné à une température comprise entre 300 et 950°C, de préférence entre 400 et 950°C et, de manière encore plus préférée, entre 450 et 800°C.

Ce procédé de préparation d'un catalyseur apte à la réaction d'isosynthèse, est un procédé du type sol-gel qui permet d'obtenir des oxydes d'au moins deux métaux dont la réactivité vis-à-vis de l'eau et du dioxyde de carbone est sensiblement nulle : ainsi, il permet de préparer des oxydes qui ne se transforment pas en hydroxydes, carbonates, hydroxycarbonates ou hydrogénocarbonates desdits métaux en présence d'eau ou/et de dioxyde de carbone, contrairement aux oxydes simples des métaux contenus dans ces oxydes préparés selon l'invention (R. ALVERO, S. BERNAL et al., Journal of Less Common Metals, 94, 139, 1983). Après l'étape 4) (étape de calcination) du procédé de préparation selon l'invention, lesdits métaux sont en effet totalement combinés entre eux à l'état d'oxyde dans une structure oxyde de type cubique, perovskite, grenat, ilménite ou fluorine ou de type apparenté au type fluorine (par exemple pyrochlore), microcristallisée.

La méthode de fabrication du catalyseur est particulièrement adaptée pour préparer un oxyde d'au moins deux métaux différents présentant les caractéristiques sus-mentionnées, l'un au moins desdits métaux étant choisi dans le groupe formé par le magnésium, le calcium, le strontium, le baryum, le lanthane, le cérium, le praséodyme, le néodyme, le samarium, le gadolinium et l'uranium, l'autre au moins desdits métaux étant choisi dans le groupe formé par le lithium, le sodium, le potassium, le rubidium, le magnésium, le calcium, le strontium, le baryum, l'yttrium, l'uranium, le titane, le zirconium, l'hafnium, le chrome, l'aluminium, le gallium, le germanium, l'étain et le plomb. A titre d'exemples, on peut citer les oxydes répondant à l'une des formules suivantes : $LaYO_3$, $La_2Zr_2O_7$, $SmAlO_3$, $Gd_3Al_5O_{12}$.

On entend par solvant approprié, utilisé dans l'étape 1) du procédé selon l'invention, un solvant dans lequel le sel ou l'oxyde simple du métal qui y est introduit est soluble.

Chacun des solvants utilisés dans cette étape 1) peut être par exemple choisi dans le groupe formé par les alcools et les acides organiques, et, notamment, dans le groupe formé par le méthanol, l'éthanol, l'acide acétique, l'acide propionique et l'acide butyrique.

Les mises en solution, lors de cette étape 1), peuvent éventuellement être effectuées en présence d'iode sublimée. Elles sont généralement effectuées sous agitation.

Chaque sel ou oxyde simple de métal utilisé lors de cette étape 1) doit être soluble dans son solvant respectif. Ledit sel est par exemple choisi parmi les nitrates, les oxynitrates, les chlorures, les oxychlorures, les sulfates, les oxysulfates, les acétates et également parmi les hydroxydes, les hydroxycarbonates et les carbonates solubles en milieu acide, les complexes aminés ou les composés organométalliques tels que les alcoxydes dudit métal. D'une manière préférée, chaque sel est choisi dans le groupe formé par les nitrates, les chlorures et les alcoxydes.

Les sels ou oxydes simples des métaux sont mis en solution, lors de l'étape 1), dans des proportions telles que, lors des étapes ultérieures du procédé selon l'invention, les oxydes simples desdits métaux peuvent totalement se combiner entre eux pour former un oxyde d'au moins deux métaux présentant une structure de type cubique, perovskite, grenat, ilménite ou fluorine ou de type apparenté au type fluorine (par exemple pyrochlore).

Lors de l'étape 2) du procédé selon l'invention, le mélange des solutions, contenant chacune l'un desdits métaux et obtenues à l'étape 1), est habituellement réalisé sous agitation.

L'évaporation de ce mélange (étape 3) du procédé selon l'invention), effectuée par exemple entre 65 et 180°C , permet l'obtention d'un gel contenant lesdits métaux; ce gel est ensuite calciné (étape 4) du procédé selon l'invention), après avoir été, par exemple, soit filtré puis lavé avec un solvant tel qu'un alcool soit recueilli après une trempe dans un bain d'azote liquide.

L'oxyde obtenu à l'issue de l'étape 4) du procédé selon l'invention présente donc la particularité de ne pas pouvoir se réhydrater ou se recarbonater à l'air ou dans un milieu liquide ou gazeux contenant de l'eau ou/et du dioxyde de carbone. Cette propriété peut être mise en évidence par analyse en diffraction des rayons X; elle peut également être mise en évidence pendant et après un test d'isosynthèse, utilisant ledit oxyde comme catalyseur, à une température comprise généralement entre 350 et 550°C (par exemple entre 400 et 500°C), à une pression comprise habituellement entre 20 et 50 MPa (par exemple entre 35 et 45 MPa) et en présence d'un mélange d'hydrogène et de monoxyde de carbone dont le rapport molaire $H_2/CO$ est généralement compris entre 0,1 et 3 (par exemple entre 0,5 et 1,5) et un débit horaire de gaz compris en général entre 0,5 et 10 l/(g de catalyseur). Ce test permet notamment d'étudier le comportement d'oxydes en présence de quantités importantes d'eau et de dioxyde de carbone dans des conditions opératoires particulièrement sévères.

Selon un autre objet de l'invention, il est également proposé une solution solide d'au moins un oxyde simple d'un métal, choisi parmi les métaux des groupes IIa (en particulier le magnésium, le calcium, le strontium, le baryum) et IIIb (en particulier l'yttrium, le lanthane, le cérium, le praséodyme, le néodyme, le samarium, le gadolinium et l'uranium) de la classification périodique des éléments, dans un oxyde obtenu suivant le procédé de préparation de l'invention.

Cette solution solide possède également la particularité d'avoir une réactivité sensiblement nulle vis-à-vis de l'eau et du dioxyde de carbone.

L'oxyde préparé comme indiqué ci-dessus, ainsi que ladite solution solide, sont utilisés chacun comme catalyseur dans un procédé d'isosynthèse.

Les exemples suivants illustrent l'invention.

<u>EXEMPLE 1</u> Préparation de l'oxyde $La_2Zr_2O_7$ de structure pyrochlore.

Une solution $S_1$ est préparée par dissolution (sous agitation et à 130°C) de 5 g de $La_2O_3$ dans 100 ml d'acide propionique en présence d'un crystal d'iode sublimée (étape 1) du procédé selon l'invention.).

Une solution $S_2$ est préparée par dissolution (sous agitation) de 13,82 g de n-propylate de zirconium dans 150 ml d'acide propionique (étape 1) du procédé selon l'invention.).

Les solutions $S_1$ et $S_2$ sont ensuite mélangées ensemble (étape 2) du procédé selon l'invention).

Puis, le mélange obtenu est évaporé à 145°C jusqu'à l'obtention d'un gel (étape 3) du procédé selon l'invention) qui est recueilli après une trempe dans un bain d'azote liquide.

Enfin, ce gel est ensuite calciné (étape 4) du procédé selon l'invention) progressivement jusqu'à 650°C (programmation de température : 1°C/mn, puis palier de 2 heures).

4

EXEMPLE 2 Préparation de l'oxyde $LaYO_3$ de structure cubique.

Une solution $S_1$ est préparée d'une manière identique à celle de l'exemple 1 (étape 1) du procédé selon l'invention).

Une solution $S_3$ est préparée par dissolution (sous agitation ) de 3,46 g de $Y_2O_3$ dans 100 ml d'acide propionique en présence d'un cristal d'iode sublimée (étape 1) du procédé selon l'invention).

Les solutions $S_1$ et $S_3$ sont ensuite mélangées ensemble (étape 2) du procédé selon l'invention).

Puis, le mélange obtenu est évaporé à 145°C jusqu'à l'obtention d'un gel (étape 3) du procédé selon l'invention ) qui est recueilli après une trempe dans un bain d'azote liquide.

Enfin, ce gel est ensuite calciné (étape 4) du procédé selon l'invention) progressivement jusqu'à 600°C (programmation de température: 1°C/mn , puis palier de 2 heures).

EXEMPLE 3 Préparation de l'oxyde $SmAlO_3$ de structure perovskite.

Une solution $S_4$ est préparée par dissolution (sous agitation) de 2,66 g de $Sm_2O_3$ dans 100 ml d'acide propionique (étape 1) du procédé selon l'invention).

Une solution $S_5$ est préparée par dissolution (sous agitation) de 3 g de $Al(n-OC_4H_9)_3$ dans 100 ml d'acide propionique (étape 1) du procédé selon l'invention).

Les solutions $S_4$ et $S_5$ sont ensuite mélangées ensemble (étape 2) du procédé selon l'invention).

Puis le mélange obtenu est évaporé à 145°C jusqu'à l'obtention d'un gel (étape 3) du procédé selon l'invention) qui est recueilli après une trempe dans un bain d'azote liquide.

Enfin, ce gel est ensuite calciné (étape 4) du procédé selon l'invention) progressivement jusqu'à 650°C (programmation de température 1°C/mn, puis palier de 5 heures).

EXEMPLE 4 Préparation de l'oxyde $Gd_3Al_5O_{12}$ de structure grenat.

Une solution $S_6$ est préparée par dissolution (sous agitation) de 2 g de $Gd_2O_3$ dans 150 ml d'acide propionique en présence d'un cristal d'iode sublimée (étape 1) du procédé selon l'invention).

Une solution $S_7$ est préparée par dissolution (sous agitation) de 4,4 g de $Al(n-OC_4H_9)_3$ dans 100 ml d'acide propionique (étape 1) du procédé selon l'invention).

Les solutions $S_6$ et $S_7$ sont ensuite mélangées ensemble (étape 2) du procédé selon l'invention).

Puis, le mélange obtenu est évaporé à 145°C jusqu'à l'obtention d'un gel (étape 3) du procédé selon l'invention) qui est recueilli après une trempe dans un bain d'azote liquide.

Enfin, ce gel est ensuite calciné (étape 4) du procédé selon l'invention) progressivement jusqu'à 650°C (programmation de température: 1°C/mn, puis palier de 4 heures).

EXEMPLE 5 (comparatif) Préparation de l'oxyde $La_2O_3$.

Une solution $S_1$ est préparée de manière identique à celle de l'exemple 1.

Cette solution $S_1$ est ensuite évaporée à 145°C jusqu'à l'obtention d'un gel qui est recueilli après une trempe dans un bain d'azote liquide.

Ce gel est ensuite calciné progressivement jusqu'à 600°C (programmation de température : 1°C/mn, puis palier de 2 heures).

EXEMPLE 6 (comparatif) Préparation d'un oxyde $La_2Zr_2O_7$.

On dissout dans 200 ml d'eau 262,9 g d'acide citrique, 21,65 g de nitrate de lanthane hexahydraté et 13,36 g de nitrate de zirconyle dihydraté. On ajoute 300 ml d'une solution d'ammoniaque de densité 0,92 à 20°C (10,85 N). On maintient à ébullition pendant 4 heures.

On évapore ensuite sous 20 mm de mercure dans un évaporateur rotatif à 75°C, jusqu'à obtention d'un sirop épais, de viscosité 2000 centipoises à 20°C, qui est ensuite déshydraté à l'étuve à vide à 80°C pendant 12 heures.

L'oxyde est obtenu par décomposition à l'air à 600°C pendant 4 heures.

EXEMPLE 7 (comparatif) Préparation d'un oxyde $Sm_2O_3$.

Une solution $S_8$ est préparée par dissolution (sous agitation) de 5g de $Sm_2O_3$ dans 200 ml d'acide propionique en présence d'un cristal d'iode sublimée.

Cette solution $S_8$ est ensuite évaporée à 145°C jusqu'à l'obtention d'un gel qui est recueilli après une trempe dans un bain d'azote liquide. Ce gel est ensuite calciné progressivement jusqu'à 600°C (programmation de température : 1°C/mn, puis palier de 2 heures).

EXEMPLE 8 Test en réaction d'isosynthèse (stabilité en présence d'eau et de dioxyde de carbone).

Les oxydes prépararés dans les exemples 1 à 7 sont chacun employés comme catalyseur d'isosynthèse. Ces tests en isosynthèse (dans lesquels se forme une quantité importante d'$H_2O$ et de $CO_2$) sont chacun réalisés dans les conditions opératoires suivantes :

| - température | 475°C; |
|---|---|
| - pression | 40 MPa; |
| - rapport molaire H2/CO | 1 ; |
| - débit horaire de gaz | 1,2 l/(g de catalyseur). |

Le tableau 1 résume les résultats catalytiques obtenus après 24 heures et 192 heures de test et le tableau 2 présente une comparaison des propriétés texturales (surface spécifique déterminée par la méthode B.E.T.) et structurales (par diffraction des rayons X) des oxydes avant et après test.

Les résultats donnés dans les tableaux 1 et 2 montrent notamment que les oxydes préparés selon l'invention (exemples 1 à 4) ont une activité stable dans le temps et une réactivité sensiblement nulle vis-à-vis de l'eau et du dioxyde de carbone, contrairement aux autres oxydes (exemples 5 à 7).

## Tableau 1

| Exemple | Formule de l'oxyde | Durée du test (heures) | Conversion % (avec R= Groupement alkyle,$CnH_{2n+1}$) | | |
|---|---|---|---|---|---|
| | | | RH | ROH+D.M.E. | $CO_2$ |
| 1 | $La_2Zr_2O_7$ | 24 | 25 | 1,6 | 22,4 |
| | | 192 | 25 | 1,8 | 24,3 |
| 2 | $LaYO_3$ | 24 | 12 | 1,4 | 20,2 |
| | | 192 | 12 | 1,4 | 22,6 |
| 3 | $SmAlO_3$ | 24 | 15 | 1,5 | 30,0 |
| | | 192 | 15 | 1,5 | 30,5 |
| 4 | $Gd_3Al_5O_{12}$ | 24 | 17 | 1,7 | 32,2 |
| | | 192 | 17 | 1,9 | 33,1 |
| 5* | $La_2O_3$ | 24 | 10 | 2 | 17,5 |
| | | 192 | 6 | 1 | 9 |
| 6* | $La_2Zr_2O_7$ | 24 | 26 | 1,6 | 23,2 |
| | | 192 | 19 | 1,2 | 17,4 |
| 7* | $Sm_2O_3$ | 24 | 12 | 2,8 | 21,7 |
| | | 192 | 8 | 1,9 | 14,8 |

avec D.M.E. = dimethyléther
* Exemples comparatifs

**Tableau 2xw**

| Exemple | Formule de l'oxyde | Surface spécifique ($m^2/g$) mesurée par B.E.T. | | Structure cristalline observée par diffraction des rayons X | |
|---|---|---|---|---|---|
| | | avant test | après test | avant test | après test |
| 1 | $La_2Zr_2O_7$ | 40 | 36 | $La_2Zr_2O_7$ | $La_2Zr_2O_7$ |
| 2 | $LaYO_3$ | 24 | 22 | $LaYO_3$ | $LaYO_3$ |
| 3 | $SmAlO_3$ | 32 | 28 | $SmAlO_3$ | $SmAlO_3$ |
| 4 | $Gd_3Al_5O_{12}$ | 47 | 43 | $Gd_3Al_5O_{12}$ | $Gd_3Al_5O_{12}$ |
| 5* | $La_2O_3$ | 17 | 2 | $La_2O_3+La(OH)_3$ | $La(OH)_3 + La_2O_2CO_3$ |
| 6* | $La_2Zr_2O_7$ | 22 | 10 | $La_2Zr_2O_7$ | $La(OH)_3+La_2Zr_2O_7+La_2O_2CO_3$ |
| 7* | $Sm_2O_3$ | 17 | 5 | $Sm_2O_3$ | $Sm(OH)_3+Sm_2O_3+Sm_2O_2CO_3$ |

\* Exemples comparatifs

## Revendications

1. Procédé d'isosynthèse en présence d'un catalyseur à base d'un oxyde d'au moins deux métaux différents, l'un au moins desdits métaux étant choisi parmi les métaux des groupes IIa et IIIb de la classification périodique des éléments, l'autre au moins desdits métaux étant choisi parmi les métaux des groupes Ia, IIa, IIIb, IVb, Vb, VIb, IIIa et IVa de la classification périodique des éléments, ledit oxyde ayant une surface spécifique d'au moins 20 $m^2/g$ et une structure de l'un des types suivants : cubique, perovskite, grenat, ilménite, fluorine ou type apparenté au type fluorine, caractérisé en ce que le catalyseur est préparé selon un procédé comprenant les étapes suivantes :

1/ un sel ou un oxyde simple de chacun desdits métaux est mis en solution dans un solvant approprié,

2/ les solutions, contenant chacune l'un desdits métaux, obtenues à l'étape 1) sont mélangées ensemble,

3/ le mélange de solutions obtenu à l'étape 2) est évaporé jusqu'à l'obtention d'un gel contenant lesdits métaux,

4/ le gel obtenu à l'étape 3) est calciné à une température comprise entre 300 et 950°C.

2. Procédé selon la revendication 1 dans lequel l'un au moins desdits métaux est choisi dans le groupe formé par le magnésium, le calcium, le strontium, le baryum, le lanthane, le cérium, le praséodyme, le néodyme, le samarium, le gadolinium et l'uranium, l'autre au moins desdits métaux étant choisi dans le groupe formé par le lithium, le sodium, le potassium, le rubidium, le magnésium, le calcium, le strontium, le baryum, l'yttrium, l'uranium, le titane, le zirconium, l'hafnium, le chrome, l'aluminium, le gallium, le germanium, l'étain et le plomb.

3. Procédé selon l'une des revendications 1 à 2 dans lequel ledit oxyde répond à l'une des formules suivantes : $LaYO_3$, $La_2Zr_2O_7$, $SmAlO_3$, $Gd_3Al_5O_{12}$.

4. Procédé selon l'une des revendications 1 à 3 dans lequel chaque solvant utilisé dans l'étape 1) est choisi dans le groupe formé par les alcools et les acides organiques.

5. Procédé selon l'une des revendications 1 à 4 dans lequel chaque solvant utilisé dans l'étape 1) est choisi dans le groupe formé par le méthanol, l'éthanol, l'acide acétique, l'acide propionique et l'acide butyrique.

6. Procédé selon l'une des revendications 1 à 5 dans lequel chaque sel est choisi dans le groupe formé par les nitrates, les oxynitrates, les chlorures, les oxychlorures, les sulfates, les oxysulfates, les acétates, les hydroxydes, les hydroxycarbonates, les carbonates, les complexes aminés et les composés organométalliques.

7. Procédé selon l'une des revendications 1 à 6 dans lequel chaque sel est choisi dans le groupe formé par les nitrates, les chlorures et les alcoxydes.

8. Procédé selon l'une des revendications 1 à 7 dans lequel, dans l'étape 4), ladite température est comprise entre 400 et 950°C.

9. Procédé d'isosynthèse en présence d'une solution solide d'au moins un oxyde simple d'un métal choisi parmi les métaux des groupes IIa et IIIb de la classification périodique des éléments dans un oxyde préparé suivant le procédé selon l'une des revendications 1 à 8.

**Claims**

1. An isosynthesis process in the presence of a catalyst based on an oxide of at least two different metals, one at least of said metals being selected from the metals of the groups IIa and IIIb of the periodic table of elements, the other one at least of said metals being selected from the metals of the groups Ia, IIa, IIIb, IVb, Vb, VIb, IIIa and IVa of the periodic table of elements, said oxide having a specific surface of at least 20 $m^2/g$ and a structure of one of the following types : cubic, perovskite, garnet, ilmenite, fluorite or a type related to the fluorite type, characterized in that the catalyst is prepared according to a process comprising the following stages :

1/ a salt or a simple oxide of each of said metals is brought into solution in an appropriate solvent,

2/ the solutions, containing each one of said metals, obtained in stage 1/ are mixed together,

3/ the mixture of solutions obtained in stage 2/ is evaporated until a gel containing said metals is obtained,

4/ the gel obtained in stage 3/ is calcined at a temperature ranging between 300°C and 950°C.

2. A process as claimed in claim 1, wherein one at least of said metals is selected from the group made up of magnesium, calcium, strontium, barium lanthanium, cerium, praseodymium, neodymium, samarium, gadolinium and uranium, the other at least of said metals being selected from the group made up of lithium, sodium, potassium, rubidium, magnesium, calcium, strontium, barium, yttrium, uranium, titanium, zirconium, hafnium, chromium, aluminum, gallium, germanium, tin and lead.

3. A process as claimed in any one of claims 1 to 2, wherein said oxide complies with one of the following formulas : $LaYO_3$, $La_2Zr_2O_7$, $SmAlO_3$, $Gd_3Al_5O_{12}$.

4. A process as claimed in any one of claims 1 to 3, wherein each solvent used in stage 1/ is selected from the group made up of the alcohols and the organic acids.

5. A process as claimed in any one of claims 1 to 4, wherein each solvent used in stage 1/ is selected from the group made up of methanol, ethanol, acetic acid, propionic acid and butyric acid.

6. A process as claimed in any one of claims 1 to 5, wherein each salt is selected from the group made up of the nitrates, the oxynitrates, the chlorides, the oxychlorides, the sulfates, the oxysulfates, the acetates, the hydroxides, the hydroxycarbonates, the carbonates, the amine complexes and the organometallic compounds.

7. A process as claimed in any one of claims 1 to 6, wherein each salt is selected from the group made up of the nitrates, the chlorides and the alkoxides.

8. A process as claimed in any one of claims 1 to 7, wherein in stage 4/, said temperature ranges between 400°C and 950°C.

9. An isosynthesis process in the presence of a solid solution of at least one simple oxide of a metal selected from the metals of the groups IIa and IIIb of the periodic table of elements in an oxide prepared according to the process as claimed in any one of claims 1 to 8.

## Patentansprüche

1. Verfahren zur Ausführung der Isosynthese in Anwesenheit eines Katalysators auf Basis eines Oxids aus wenigstens zwei Verschiedenen Metallen, wobei wengistens eines der Metalle aus den Metallen der Gruppen IIa und IIIb des Periodensystems ausgewählt ist, das andere wenigstens der Metalle aus den Metallen der Gruppen Ia, IIa, IIIb, IVb, Vb, VIb, IIIa und IVa des Periodensystems ausgewählt ist, wobei das Oxid eine spezifische Oberfläche von wenigstens 20 $m^2/g$ und eine Struktur einer der folgenden Typen aufweist: kubisch, Perovskit, Granat, Ilmenit, Fluorit oder einem Typ der dem Fluorit-Typ ähnlich ist, dadurch gekennzeichnet, daß der Katalysator gemäß einem Verfahren hergestellt wird, das die folgenden Schritte umfaßt:

1) ein Salz oder ein einfaches Oxid jedes der Metalle wird in einem geeigneten Lösungsmittel in Lösung gebracht,
2) die in Schritt 1) erhaltenen Lösungen, weiche jede eines der Metalle enthalten, werden zusammen vermischt,
3) die in Schritt 2) erhaltene Mischung der Lösungen wird bis zum Erhalten eines Gels, das die Metalle enthält, eingedampft,
4) das in Schritt 3) erhaltene Gel wird bei einer Temperatur, die zwischen 300 und 950°C liegt, kalziniert.

2. Verfahren nach Anspruch 1, worin wenigstens eines der Metalle aus der Gruppe ausgewählt ist, die aus Magnesium, Kalzium, Strontium, Barium, Lanthan, Cer, Praseodym, Neodym, Samarium, Gadolinium und Uran gebildet ist, wobei das andere wenigstens der Metalle aus der Gruppe ausgewählt ist, die durch Lithium, Natrium, Kaiium, Rubidium, Magnesium, Kalzium, Strontium, Barium, Yttrium, Uran, Titan, Zirkon, Hafnium, Chrom, Aluminium, Gallium, Germanium, Zinn und Blei gebildet ist.

3. Verfahren nach einem der Ansprüche 1 bis 2, worin das Oxid einer der folgenden Formeln entspricht: $LaYO_3$, $La_2Zr_2O_7$, $SmAlO_3$, $Gd_3Al_5O_{12}$.

4. Verfahren nach einem der Ansprüche 1 bis 3, worin jedes in Schritt (1) verwendete Lösungsmittel aus der Gruppe ausgewählt ist, die durch die Alkohole und die organischen Säuren gebildet ist.

5. Verfahren gemäß einem der Ansprüche 1 bis 4, worin jedes in Schritt (1) verwendete Lösungsmittel aus der Gruppe ausgewählt ist, die durch Methanol, Ethanol, Essigsäure, Propionsäure und Buttersäure gebildet ist.

6. Verfahren gemäß einem der Ansprüche 1 bis 5, worin jedes Salz aus der Gruppe ausgewählt ist, die durch die Nitrate, Oxinitrate, Chloride, Oxichloride, Sulfate, Oxisulfate, Acetate, Hydroxide, Hydroxicarbonate, Carbonate, den aminierten Komplexen und den organometallischen Verbindungen gebildet ist.

7. Verfahren nach einem der Ansprüche 1 bis 6, worin jedes Salz aus der Gruppe ausgewählt ist, die durch die Nitrate, die Chloride und die Alkoxide gebildet ist.

8. Verfahren nach einem der Ansprüche 1 bis 7, worin in Schritt 4) die Temperatur zwischen 400 und 950°C liegt.

9. Verfahren zur Ausführung der Isosynthese in Anwesenheit einer festen Lösung wenigstens eines einfachen Oxides eines Metalles, das aus den Metallen der Gruppen IIa und IIIb des Periodensystems ausgewählt ist, in einem Oxid, das folgend dem Verfahren gemäß einem der Ansprüche 1 bis 8 hergestellt ist.